(19) Europäisches Patentamt
European Patent Office
Office européen des brevets

(11) Publication number: 0 094 632 B1

(12) **EUROPEAN PATENT SPECIFICATION**

(45) Date of publication of patent specification: 26.02.86

(51) Int. Cl.[4]: C 07 C 129/12, A 61 K 31/16, C 07 D 309/12

(21) Application number: 83104712.1

(22) Date of filing: 13.05.83

(54) **(-)-15-Deoxyspergualin, a process for the preparation of the same, and a pharmaceutical composition containing the same.**

(30) Priority: 17.05.82 JP 81398/82

(43) Date of publication of application: 23.11.83 Bulletin 83/47

(45) Publication of the grant of the patent: 26.02.86 Bulletin 86/09

(84) Designated Contracting States: AT BE CH DE FR GB IT LI LU NL SE

(56) References cited:

CHEMICAL ABSTRACTS, vol. 96, no. 13, March 29, 1982, page 383, abstract no. 100578v COLUMBUS OHIO (US) T. TAKEUCHI et al.: "A new antitumor antibiotic, spergualin: isolation and antitumor activity".

CHEMICAL ABSTRACTS, vol. 98, no. 15, April 11, 1983, page 603, abstract no. 125712p COLUMBUS OHIO (US) H. IWASAWA et al.: "Synthesis of (-)-15-deoxyspergualin and (-)-spergualin-15-phosphate".

(73) Proprietor: MICROBIAL CHEMISTRY RESEARCH FOUNDATION
14-23, Kamiosaki 3 Chome Shinagawa-ku
Tokyo 141 (JP)

(72) Inventor: Umezawa, Hamao, Prof.
4-23, Toyotama-kita Nerima-ku
Tokyo (JP)
Inventor: Kondo, Shin-ichi
1157-1, Ichigao Midori-ku
Yokohama-City Kanagawa Prefecture (JP)
Inventor: Takeuchi, Tomio
5-1-11, Higashi-Gotanda
Shinagawa-ku Tokyo (JP)

(74) Representative: Reuter, Johann-Heinrich, Dr. et al
HOECHST AKTIENGESELLSCHAFT Zentrale
Patentabteilung Postfach 80 03 20
D-6230 Frankfurt/Main 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to (−)-15-deoxyspergualin of the following formula (I)

```
                         (−)
        15 14 13 12 11 10 9  8   7   6   5   4  3   2   1
H2NCNH(CH2)4CH2CH2CONHCHCONHCH2CH2CH2CH2NHCH2CH2CH2NH2          (I)
    ||                  |
    NH                  OH
```

and its salts, a process for the preparation of these compounds, and intermediates of these compounds.

In connection with histological studies on carcinostatic agents, the novel carcinostatic antibiotic BMG 162-aF2, later named spergualin, was discovered in a broth for culture of Bacillus laterosporus BMG 162-aF2 (Deposit No. 5230 at the Fermentation Research Institute, Agency of Industrial Science and Technology, Ministry of International Trade and Industry of Japan), a strain belonging to the genus *Bacillus* (Journal of Antibiotics, Vol. 34, 1619—1622, 1981). The chemical structure of spergualin is expressed by the following formula

```
                               15
          19 18 17 16 (S)14 13 12 11 10 9  8   7   6   5  4  3   2   1
H2NCNHCH2CH2CH2CH2CHCH2CONHCHCONHCH2CH2CH2CHNHCH2CH2CH2NH2
    ||                |        |
    NH                OH       OH
```

in which the configuration at the 15-position is sinistral, but that at the 11-position has not been determined (Journal of Antibiotics, Vol. 34, 1622, 1981). The compound of this structural formula could also be synthesized by the condensation of an acid amide and glyoxylylspermidine, and the resulting epimer was divided into (−)-spergualin that naturally occurs and (+)-spergualin that does not (Journal of Antibiotics, Vol. 34, 1625, 1981). Now it has been found that the optically active compound (−)-15-deoxyspergualin of the aforementioned general formula (I) produced by deoxidation of (−)-spergualin has a markedly better carcinostatic action that 15-deoxyspergualin, a compound synthesized previously through the condensation of (S)-7-guanidino-3-hydroxyheptanamide and N-[4-(3-aminopropyl)aminobutyl]-2,2-dihydroethanamide.

Since (−)-15-deoxyspergualin according to this invention is unstable in the form of a free base, it should preferably be converted into a non-toxic salt through an addition reaction with a pharmacologically acceptable acid by a customary method.

Example of the acid-addition salt include salts with inorganic acids such as hydrochloric acid, sulfuric acid, phosphoric acid and boric acid, and salts with organic acids such as acetic acid, citric acid, tartaric acid and glutaric acid.

The physical and chemical properties and biological properties of (−)-15-deoxyspergualin of the general formula (I) in accordance with this invention are revealed below.

(1) Physical and chemical properties of (−)-15-deoxyspergualin trihydrochloride dihydrate

The compound was colorless and syrupy, and its melting point could not be measured clearly. It showed $[\alpha]_D^{25}-7.3°$ (c: 1, water). Its elementary analysis revealed C: 38.45%, H: 8.08%, N: 18.61%, Cl: 20.26%, which corresponded with the theory for $C_{17}H_{37}N_7O_3 \cdot 3HCl \cdot 2H_2O$ (C: 38.31%, H: 8.32%, N: 18.40%, Cl: 19.96%). Thin-layer chromatography over silica gel using a butanol:pyridine:acetic acid:water (6:4:2:4 in volume ratio) mixture as a developer revealed a single spot (ninhydrin color reaction) at an Rf value of 0.17 (spergualin has an Rf value of 0.13). The antibacterial activity against Bacillus subtilis PCI-219 was 134% of that of (−)-spergualin ($3HCl \cdot 1/2H_2O$).

(2) Biological properties of (−)-15-deoxyspergualin trihydrochloride dihydrate

10.5 Mouse Leukemia L1210 cells were inoculated intraperitoneally to groups of 8 mice each. The test compound dissolved in physiological saline solution was intraperitoneally administered once daily for 9 consecutive days, starting on the day inoculation. The animals were raised and observed for 60 days to determine survival rates (%). The survival rates were calculated from the following equation:

$$\frac{\text{T[average period (in days) of survival for the treatment group]}}{\text{C[average period (in days) for survival for the non-treatment group]}} \times 100$$

The average survival period for the control (non-treatment group was 7.6 to 8.9 days).
The test results are shown in Table 1 along with those on (−)-spergualin.

TABLE 1

Efficacy of (−)-15-deoxyspergualin in the treatment of mouse Leukemia L1210

| Dose (mg/kg/day) | (−)-15-Deoxyspergualin* T/C (%) | No. of mice that survived 60 days | (−)-Spergualin** T/C% | No. of mice that survived 60 days |
|---|---|---|---|---|
| 50 | | | 295 | 0/8 |
| 25 | | | 334 | 0/8 |
| 12.5 | | | >586 | 4/8 |
| 6.25 | 408 | 0/8 | >732 | 8/8 |
| 3.13 | >526 | 2/8 | >441 | 3/8 |
| 1.56 | >493 | 2/8 | >301 | 1/8 |
| 0.78 | >789 | 8/8 | 107 | 0/8 |
| 0.39 | >629 | 6/8 | | |
| 0.20 | >664 | 6/8 | | |
| 0.10 | 138 | 0/8 | | |
| 0.05 | 129 | 0/8 | | |

Notes: *Trihydrochloride dihydrate
**Trihydrochloride hemihydrate

(−)-15-deoxyspergualin according to this invention is synthesized in the way mentioned below.

The hydroxyl group at the 15-position of a derivative of (−)-spergualin expressed by the following general formula (II)

```
                                      (−)
     19 18 17 16 15 14 13 12 11 10 9 8 7 6 5 4 3 2 1
H2NCNHCH2CH2CH2CH2CHCH2CONHCHCONHCH2CH2CH2CH2NCH2CH2CH2—R1      (II)
   ||              |         |                  |
   NH              OH        OR3                R2
```

wherein $R_1$ represents a masked amino group, and $R_2$ and $R_3$ each represent a masking group is deoxidized, and then, the masking groups are eliminated to afford the instantly claimed (−)-15-deoxyspergualin of the following formula (I)

```
                    (−)
H2NCNH(CH2)6CONHCHCONH(CH2)4NH(CH2)3NH2      (I)
   ||             |
   NH             OH
```

or its acid-addition salts. The amino group at the 1-position and the amino group at the 4-position in (−)-spergualin derivatives of the general formula (II) may be masked with amino-masking groups so far widely used in the synthesis of peptides. Since the compound of the formula (I) to be produced in this invention is very unstable to alkalis and acids, however, the masking should preferably be done using aralkyloxycarbonyl groups, such as benzyloxycarbonyl group and a p-methoxybenzyloxycarbonyl group, which can be easily eliminated by customary-manner hydrogenolysis. To introduce these amino-masking groups in (−)-spergualin, it is advantageous to use known methods, such as the active ester method. Generally, with these methods, these amino-masking groups do not react with the guanidine group of (−)-spergualin. The masking group for the hydroxy group at the 11-position may be any group which can be introduced without isomerizing the compound, but it should preferably be a tetrahydropyranyl group. To attach a tetrahydropyranyl group selectively to the hydroxyl group at the 11-position without isomerizing the compound, 1 to 3 equivalents of 2,3-dihydro-4H-pyran is reacted with the compound in an

anhydrous organic solvent, preferably, anhydrous dimethylformamide for 2 to 10 hours at room temperature in the presence of 0.1 to 3 equivalents, preferably, 0.2 to 1 equivalent, of an acid catalyst such as p-toluenesulfonic acid. Preferably, the reaction with 2 equivalents of 2,3-dihydro-4H-pyran is performed for 7 hours in the presence of 0.5 equivalent of p-toluenesulfonic acid, whereby a tetrahydropyranyl group can be attached selectively to the hydroxy group at the 11-position without isomerizing the compound.

The resulting compound can be expressed by the following general formula (III)

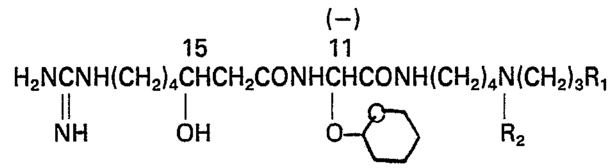

(III)

in which $R_1$ and $R_2$ are the same as defined earlier. The configuration at the 11-position of the so obtained (−)-1-N,4-bis-(benzyloxycarbonyl)-11-O-tetrahydropyranyl-spergualin has been confirmed to undergo no isomerization, in view of, say, the fact that (−)-spergualin with high optical purity was recovered by removing the amino-masking groups through hydrogenolysis and eliminating the tetrahydropyranyl group through hydrolysis with a weak acid to be described later. The tetrahydropyranyl group is a mixture of the α-body and the β-body.

Deoxidation of the hydroxyl group at the 15-position of the (−)-spergualin derivative of the general formula (II) can be performed in a customary manner. For example, a method may be employed which comprises esterifying the hydroxyl group at the 15-position with sulfonic acid, then iodinating or brominating the compound, and dehalogenating it through catalytic reduction. More concretely, the method converts the hydroxyl group at the 15-position into a sulfonic acid ester by treating the (−)-spergualin derivative of the general formula (II) with a widely used sulfonyl compound, e.g., an alkylsulfonyl compound such as methanesulfonyl chloride, an arylsulfonyl compound such as p-toluenesulfonyl chloride, or an arylalkylsulfonyl compound such as benzylsulfonyl chloride, in a solvent such as anhydrous pyridine.

Then, the resulting compound is reacted with a halogenating agent such as an alkali metal iodide or alkali metal bromide (e.g. sodium iodide or sodium bromide) in a solvent such as anhydrous N,N-dimethylformamide to obtain its derivative iodinated or brominated at the 15-position. Subsequently, the derivative is catalytically reduced in a single solvent such as methanol, dioxane or water or a mixed solvent of these liquids by a customary method with the use of a catalyst such as palladium or platinum to cause dehalogenation, thereby achieving deoxidation at the 15-position. If, in this case, the amino-masking groups are aralkyloxycarbonyl groups, the catalytic reduction also detaches these groups. The 15-deoxy-11-O-tetrahydropyranyl group is easily eliminated by adding about 0.1 equivalent of p-toluenesulfonic acid to an aqueous solution of 2 equivalents of an acid-addition salt of this compound, and stirring the mixture while cooling it with ice. The reaction time should sufficiently be 5 to 7 hours. Purification of (−)-15-deoxyspergualin obtained in this invention should preferably be performed by column chromatography over a cation exchange resin using a carboxyl group as the active group. It is recommended, for example, to absorb the compound to a column packed with CM-Sephadex(R) C-25 (a product of Pharmacia, Sweden) equilibrated with 0.4 M sodium chloride, and subject the absorbate to gradient elution involving 0.4 M to 1.0 M sodium chloride. The eluate is concentrated and dried, and extracted with anhydrous methanol. The extract is applied to a column with a molecular sieve such as Sephadex(R) LH-20 (a product of Pharmacia, Sweden), and then eluted with hydrous methanol for desalting. The eluate is concentrated and dried to obtain the desired trihydrochloride of (−)-15-deoxyspergualin. Any desired addition salt is obtained depending on the type of a salt used in gradient elution from the CM-Sephadex(R) column. This invention will be described in greater detail by reference to the following examples.

Example 1

Synthesis of (−)-15-deoxyspergualin

(a) (−)-1-N,4-bis-(benzyloxycarbonyl)spergualin:

3.0 g (5.85 mmols) of (−)-15-spergualin trihydrochloride was dissolved in 30 ml of methanol, and 7.2 ml (17.6 mmols) of triethylamine was added to the solution. To the mixture was added a solution of 3.21 g (12.9 mmols) of N-benzyloxycarbonyl-oxysuccinimide in 8 ml of dioxane. The resulting mixture was stirred for 3 hours at room temperature. The reaction mixture was concentrated and dried, and the resulting concentrate was dissolved in 50 ml of 0.1 M sodium chloride.

The solution was adjusted to a pH of 6.5 with 2N hydrochloric acid, and applied to a column of CM-Sephadex(R) C-25 (200 ml) equilibrated with 0.1 M sodium chloride. Then, gradient elution using 0.1 M and 0.5 M sodium chloride (1 liter each) was performed, to collect fractions of 20 ml each. Fraction Nos. 34 to 80 were put together, concentrated and dried, and the concentrate was extracted 3 times with 10 ml of methanol. The extract was applied to a column of Sephadex(R) LH-20 (200 ml), and eluted with 90% methanol for desalting, to collect fractions of 2 ml each. Fraction Nos. 51 to 63 were put together, concentrated and dried to obtain 3.8 of (−)-1-N,4-bis(benzyloxycarbonyl) spergualin hydrochloride as a

colorless syrupy material. Yield: 91% $[\alpha]_D^{21}-11°$ (c: 1, water). Found on elementary analysis: C: 54.95%, H: 7.25%, N: 13.83%, Cl: 5.06%. Theory for $C_{33}H_{49}N_7O_8 \cdot HCl \cdot 1/2H_2O$: C: 55.26%, H: 7.17%, N: 13.67%, Cl: 4.94%.

(b) (−)1-N,4-bis-(benzyloxycarbonyl)-11-O-tetrahydropyranyl-spergualin:

3.45 g (4.81 mM) of the (−)-1-N,4-bis-(benzyloxycarbonyl)-spergualin hydrochloride obtained in the above step (a) was dissolved in 30 ml of anhydrous N,N-dimethylformamide. To the solution were added 0.63 ml (9.75 mM) of 2,3-dihydro-4H-pyran and 464 mg (2.44 mM) of p-toluenesulfonic acid ($H_2O$), and the mixture was stirred for 7 hours at room temperature. After the reaction, 0.33 ml (2.44 mM) of triethylamine was added, and the mixture was concentrated and dried. The resulting concentrated was purified by column chromatography using a column of silica gel (Wako Gel(R) C-200, 300 g) and a chloroform:methanol:pyridine:50% acetic acid (240:40:4:1) mixture as a developer. Each fraction was collected in an amount of 20 ml.

Fraction Nos. 66 to 78 were gathered, concentrated and dried to obtain 1.07 g of (−)-1-N,4-bis-(benzyloxycarbonyl)-11-O-tetrahydropyranylspergualin acetate as a colorless syrupy material. Yield: 26% $[\alpha]_D^{22}-13°$ (c: 1, methanol). Elementary analysis values: C: 56.65%, H: 7.76%, N: 11.75%. Theoretical values for $C_{38}H_{57}N_7O_9 \cdot CH_3COOH \cdot 3/2\ H_2O$: C: 56.99%, H: 7.65%, N: 11.63%. Further, Fraction Nos. 85 to 92 were gathered, concentrated and dried to recover (−)-1-N,4-bis-(benzyloxycarbonyl) spergualin acetate in an amount of 362 mg (yield: 10%).

(c) (−)-1-N,4-bis-(benzyloxycarbonyl)-15-O-methanesulfonyl-11-O-tetrahydropyranylspergualin:

950 mg (1,13 mM) of the (−)-1-N,4-bis-(benzyloxycarbonyl)-11-O-tetrahydropyranylspergualin acetate obtained in the above step (b) was dissolved in 10 ml of anhydrous pyridine. 0.13 ml (1.75 mM) of methanesulfonyl chloride was added with the solution cooled with ice, and the mixture was stirred for 3 hours. After the reaction, 0.2 ml of water was added, and the mixture was concentrated and dried. The concentrate was purified by column chromatography over silica gel (Wako Gel(R) C-200, 100 g) developed with a chloroform:methanol:pyridine:50% acetic acid (320:40:4:1) mixture. The amount of each fraction was 20 ml. Fraction Nos. 16 to 25 were gathered, concentrated and dried to obtain 598 mg of an acetate of the indicated compound as a colorless syrupy material. Its yield was 54%.

(d) (−)-1-N,4-bis-(benzyloxycarbonyl)-15-deoxy-15-iodo-11-O-tetrahydropyranylspergualin:

591 mg (0.699 mM) of the (−)-1-N,4-bis-(benzyloxycarbonyl)-15-O-methanesulfonyl-11-O-tetrahydropyranylspergualin acetate obtained in step (c) was dissolved in 20 ml of anhydrous N,N-dimethylformamide. 5.02 g (33.5 mM) of sodium iodide was added, followed by stirring the mixture for 15 hours at 90°C. The reaction mixture was concentrated and dried, and the concentrate was dissolved in 30 ml of ethyl acetate. The solution was washed with 30 ml of a 20% aqueous solution of sodium thiosulfate and 30 ml of a saturated aqueous solution of sodium chloride. The ethyl acetate layer was dehydrated with anhydrous sodium sulfate, and concentrated and dried. The concentrate was purified by a column chromatography packed with silica gel (Wako Gel(R) C-200, 50 g) and developed with a 320:40:4:1 mixture of chloroform, methanol, pyridine and 50% acetic acid. The amount of each fraction collected was 10 ml. Fraction Nos. 7 to 28 were gathered, concentrated and dried to obtain 204 mg of an acetate of the indicated compound as a colourless syrupy material. The yield was 33%.

(e) (−)-15-deoxy-11-O-tetrahydropyranylspergualin:

198 mg (0.214 mM) of the (−)-1-N,4-bis-(benzyloxycarbonyl)-15-deoxy-15-iodo-11-O-tetrahydropyranylspergualin acetate obtained in the step (d) was dissolved in 20 ml of an 80% aqueous solution of methanol. 40 mg of a catalyst consisting of 5% palladium and barium carbonate was added, and the mixture was stirred in a hydrogen stream for 10 hours at room temperature. The catalyst was removed by filtration, and the filtrate was concentrated and dried. The resulting concentrate was dissolved in 30 ml of a solution of 0.1M sodium chloride, and the solution was adjusted to a pH of 6.5 with 1N hydrochloric acid. Then, the solution was applied to a column of CM-Sephadex(R) C-25 (a product of Pharmacia, Sweden, 40 ml) equilibrated with 0.1M sodium chloride. Further, it was subjected to gradient elution involving 0.1M and 0.8M sodium chloride (200 ml each) to collect fractions in an amount of 4 ml each. Fraction Nos. 67 to 77 were gathered, concentrated and dried, and extracted 3 times with 5 ml of methanol. The extract was applied to a column of Sephadex(R) LH-20 (100 ml), and eluted with 90% methanol for desalting. The amount of each fraction was 1 ml. Fraction Nos. 36 to 47 were put together, concentrated and dried to obtain 62.9 mg of a trihydrochloride of the indicated compound as a colorless syrupy material. Its yield was 54%.

(f) (−)-15-deoxyspergualin:

61 mg (0.112 mM) of the 15-deoxy-11-O-tetrahydropyranyl-spergualin trihydrochloride obtained in the step (e) was dissolved in 3 ml of water. With the solution cooled with ice, 2.1 mg (0.011 mM) of p-toluenesulfonic acid ($H_2O$) was added, and the mixture was stirred for 7 hours. After the reaction, the mixture was adjusted to a pH of 6.5 with 1M aqueous ammonia, and 10 ml of 0.4 M sodium chloride was added. The mixture was applied to a column of CM-Sephadex(R) C-25 (20 ml) equilibrated with 0.4M sodium

chloride, and subjected to gradient elution involving 0.4M and 1.0M sodium chloride (100 ml each). The amount of each fraction collected was 2 ml. Fraction Nos. 73 to 81 were gathered, concentrated and dried, and extracted 3 times with 5 ml of methanol. The extract was applied to a column of Sephadex[(R)] LH-20 (100 ml), and eluted with 90% methanol for desalting. The amount of each fraction was 1 ml. Fraction Nos. 38 to 48 were gathered, concentrated and dried to obtain 46.6 mg of (−)-15-deoxyspergualin trihydrochloride dihydrate as a colorless syrupy material. The yield was 78%.

**Claims for the Contracting States: BE CH DE FR GB IT LI LU NL SE**

1. (−)-15-Deoxyspergualin expressed by the following formula I

(−)
15 14 13 12 11 10 9 8 7 6 5 4 3 2 1

$$H_2NCNH(CH_2)_4CH_2CH_2CONHCHCONHCH_2CH_2CH_2CH_2NHCH_2CH_2CH_2NH_2$$

|| NH (at C); | OH (at CH)

and its salts.

2. A process for preparing (−)-15-deoxyspergualin of the formula I

(−)

$$H_2NCNH(CH_2)_6CONHCHCONH(CH_2)_4NH(CH_2)_3NH_2 \qquad (I)$$

|| NH (at C); | OH (at CH)

and its salts, which comprises deoxidising the hydroxyl group at the 15-position of a derivative of (−)-spergualin expressed by the following general formula II

(−)
19 18 17 16 15 14 13 12 11 10 9 8 7 6 5 4 3 2 1

$$H_2NCNHCH_2CH_2CH_2CH_2CHCH_2CONHCHCONHCH_2CH_2CH_2CH_2NCH_2CH_2CH_2{-}R_1 \qquad (II)$$

|| NH; | OH; | $OR_3$; | $R_2$

wherein $R_1$ represents a masked amino group, and $R_2$ and $R_3$ each represent a masking group, and then eliminating the masking group.

3. A process as claimed in claim 2, wherein $R_3$ is the tetrahyhdropyranyl group.

4. A pharmaceutical composition comprising as active ingredient a compound of the formula I as claimed in claim 1 or one of its physiologically acceptable salts in association with a pharmaceutically acceptable carrier and/or stabilizer.

5. A compound as claimed in claim 1 for use as medicament.

**Claims for the Contracting State: AT**

1. A process for preparing (−)-15-deoxyspergualin of the formula

(−)

$$H_2NCNH(CH_2)_6CONHCHCONH(CH_2)_4NH(CH_2)_3NH_2 \qquad (I)$$

|| NH (at C); | OH (at CH)

and its salts, which comprises deoxidizing the hydroxyl group at the 15-position of a derivative of (−)-spergualin expressed by the following general formula

(−)
19 18 17 16 15 14 13 12 11 10 9 8 7 6 5 4 3 2 1

$$H_2NCNHCH_2CH_2CH_2CH_2CHCH_2CONHCHCONHCH_2CH_2CH_2CH_2NCH_2CH_2CH_2{-}R_1 \qquad (II)$$

|| NH; | OH; | $OR_3$; | $R_2$

wherein $R_1$ represents a masked amino group, and $R_2$ and $R_3$ each represent a masking group, and then eliminating the masking groups.

2. A process as claimed in claim 1 in which $R_3$ in formula II represents the tetrahydropyranyl group.

3. A process for the preparation of a pharmaceutical composition, characterized in that a compound of

the formula I as claimed in claim 1 and/or one of its physiologically acceptable salts, optionally in admixture or conjunction with a pharmaceutically suitable carrier and/or stabilizer is brought into in form suitable for therapeutic use.

**Patentansprüche für die Vertragsstaaten: BE CH DE FR GB IT LI LU NL SE**

1. (−)-15-Desoxyspergualin entsprechend der folgenden Formel I

(−)
15 14 13 12 11 10 9 8 7 6 5 4 3 2 1

$$H_2NCNH(CH_2)_4CH_2CH_2CONHCHCONHCH_2CH_2CH_2CH_2NHCH_2CH_2CH_2NH_2$$

||  NH (at C of guanidine); | OH (at C-11)

und dessen Salze.

2. Verfahren zur Herstellung von (−)-15-Desoxyspergualin der Formel I

(−)

$$H_2NCNH(CH_2)_6CONHCHCONH(CH_2)_4NH(CH_2)_3NH_2 \qquad (I)$$

|| NH; | OH

und dessen Salzen, dadurch gekennzeichnet, dass man die Hydroxylgruppe in 15-Stellung eines (−)-Spergualinderivats entsprechend der folgenden allgemeinen Formel II

(−)
19 18 17 16 15 14 13 12 11 10 9 8 7 6 5 4 3 2 1

$$H_2NCNHCH_2CH_2CH_2CH_2CHCH_2CONHCHCONHCH_2CH_2CH_2CH_2NCH_2CH_2CH_2—R_1 \qquad (II)$$

|| NH; | OH; | $OR_3$; $R_2$

worin $R_1$ für eine geschütze Aminogruppe sowie $R_2$ und $R_3$ je für eine Schutzgruppe stehen, desoxydiert und dann die Shutzgruppe abspaltet.

3. Verfahren nach Anspruch 2, dadurch gekennzeichnet, dass $R_3$ die Tetrahydropyranylgruppe bedeutet.

4. Pharmazeutische Zusammensetzung, dadurch gekennzeichnet, dass sie als Wirkstoff eine Verbindung der Formel I nach Anspruch 1 oder eines von deren physiologisch unbedenklichen Salzen zusammen mit einem pharmazeutisch unbedenklichen Träger und/oder Stabilisator enthält.

5. Verbindung nach Anspruch 1 zur Verwendung als Arzneimittel.

**Patentansprüche für den Vertragsstaat: AT**

1. Verfahren zur Herstellung von (−)-15-Desoxyspergualin der Formel

(−)

$$H_2NCNH(CH_2)_6CONHCHCONH(CH_2)_4NH(CH_2)_3NH_2 \qquad (I)$$

|| NH; | OH

und dessen Salzen, dadurch gekennzeichnet, dass man die Hydroxylgruppe in 15-Stellung eines (−)-Spergualinderivats entsprechend der folgenden allgemeinen Formel

(−)
19 18 17 16 15 14 13 12 11 10 9 8 7 6 5 4 3 2 1

$$H_2NCNHCH_2CH_2CH_2CH_2CHCH_2CONHCHCONHCH_2CH_2CH_2CH_2NCH_2CH_2CH_2—R_1 \qquad (II)$$

|| NH; | OH; | $OR_3$; | $R_2$

worin $R_1$ für eine geschützte Aminogruppe sowie $R_2$ und $R_3$ je für eine Schutzgruppe stehen, desoxydiert und dann die Schutzgruppen abspaltet.

2. Verfahren nach Anspruch 1, dadurch gekennzeichnet, dass $R_3$ in der Formel II die Tetrahydropyranylgruppe bedeutet.

3. Verfahren zur Herstellung einer pharmazeutischen Zusammensetzung, dadurch gekennzeichnet,

dass man eine Verbindung der Formel I nach Anspruch 1 und/oder eines von deren physiologisch unbedenklichen Salzen, gegebenenfalls im Gemisch oder in Verbindung mit einem pharmazeutisch geeigneten Träger und/oder Stabilisator, in eine zur therapeutischen Verwendung geeignete Form bringt.

**Revendications pour les Etats Contractants: BE CH DE FR GB IT LI LU NL SE**

1. (−)-15-désoxyspergualine répondant à la formule suivante:

(−)
15 14 13 12 11 10 9 8 7 6 5 4 3 2 1
$H_2NCNH(CH_2)_4CH_2CH_2CONHCHCONHCH_2CH_2CH_2CH_2NHCH_2CH_2CH_2NH_2$
|| |
NH OH

et ses sels.

2. Procédé pour la préparation de (−)-15-désoxyspergualine de formule I

(−)
$H_2NCNH(CH_2)_6CONHCHCONH(CH_2)_4NH(CH_2)_3NH_2$ (I)
|| |
NH OH

et de ses sels, qui consiste à désoxyder le groupe hydroxyle en position 15 d'un dérivé de (−)spergualine répondant à la formule générale suivante II

(−)
19 18 17 16 15 14 13 12 11 10 9 8 7 6 5 4 3 2 1
$H_2NCNHCH_2CH_2CH_2CH_2CHCH_2CONHCHCONHCH_2CH_2CH_2CH_2NCH_2CH_2CH_2—R_1$ (II)
|| | | |
NH OH $OR_3$ $R_2$

dans laquelle $R_1$ représente un groupe amino protégé, et $R_2$ et $R_3$ représentent chacun un groupe protecteur, puis à éliminer le groupe protecteur.

3. Procédé selon la revendication 2, dans lequel $R_3$ est le groupe tétrahydropyrannyle.

4. Composition pharmaceutique comprenant en tant qu'ingrédient actif un composé de formule I selon la revendication 1, ou un de ses sels physiologiquement acceptables, associé avec un véhicule et ou un stabilisant pharmacologiquement acceptable.

5. Composé selon la revendication 1, utilisable comme médicament.

**Revendication pour l'Etat Contractant: AT**

1. Procédé pour la préparation de (−)-15-désoxyspergualine répondant à la formule

(−)
$H_2NCNH(CH_2)_6CONHCHCONH(CH_2)_4NH(CH_2)_3NH_2$ (I)
|| |
NH OH

et de ses sels, qui consiste à désoxyder le groupe hydroxyle en position 15 d'un dérivé de (−)spergualine répondant à la formule générale suivante:

(−)
19 18 17 16 15 14 13 12 11 10 9 8 7 6 5 4 3 2 1
$H_2NCNHCH_2CH_2CH_2CH_2CHCH_2CONHCHCONHCH_2CH_2CH_2CH_2NCH_2CH_2CH_2—R_1$ (II)
|| | | |
NH OH $OR_3$ $R_2$

dans laquelle $R_1$ représente un groupe amino protége et $R_2$ et $R_3$ représentent chacun un groupe protecteur, puis à éliminer le groupe protecteur.

2. Procédé selon la revendication 1, dans lequel $R_3$ dans la formule II représente le groupe tétrahydropyranyle.

3. Procédé pour la préparation d'une composition pharmaceutique caractérisé en ce qu'un composé de formule I selon la revendication 1 et/ou un de ses sels physiologiquement acceptables, éventuellement mélangé ou associé avec un véhicule et/ou un stabilisant pharmaceutiquement acceptable, est mis sous une forme appropriée pour une application thérapeutique.